# EUROPEAN PATENT SPECIFICATION

(11) **EP 2 334 340 B1**
(45) Date of publication and mention of the grant of the patent: **28.08.2013**
(21) Application number: 09809355.2
(22) Date of filing: 01.09.2009
(51) Int. Cl.: A61L 2/10, A61M 39/10

(54) **ASSEMBLY AND METHOD FOR DISINFECTING LUMENS OF MEDICAL DEVICES**
VORRICHTUNG ZUM STERILISIEREN VON ROHRLUMEN MEDIZINISCHER GERÄTE
APPAREIL POUR LA STÉRILISATION DE LUMIÈRES DE TUBES DE DISPOSITIF MEDICAUX

(30) Priority: 01.09.2008 EP 08163416; 01.09.2008 US 93437 P; 08.10.2008 DK 200801417
(43) Date of publication of application: 22.06.2011
(73) Proprietor: U-VIVO ApS, 4000 Roskilde (DK)
(72) Inventor: BAK, Jimmy, DK-2670 Greve (DK)
(74) Representative: Zacco Denmark A/S
(86) International application number: PCT/EP2009/061286
(87) International publication number: WO 2010/023329

(56) References cited:
- WO-A-02/102421
- WO-A-2008/014437
- US-A1- 2007 176 117
- US-A1- 2008 159 908
- US-B1- 6 461 569

## Description

### The technical field

The invention relates to an assembly for sterilizing surfaces and lumens of a medical device with a light source. The assembly comprises a medical device having a lumen and a coupling portion, and a light source configured to emit light having a corresponding coupling portion and a separate unit which is not transparent for light from the light source. The light source can emit light that reduces the number of or removes micro organisms from the lumen and/or surfaces of the medical device.

Intravascular catheters are indispensable in modern-day medical practice, particularly in intensive care units (ICUs). Catheter-related bloodstream infections (CRBSI) resulting from bacterial colonisation of an intravascular catheter are a significant clinical problem, magnified in recent years by the increasing use of intravascular catheters in intensive care, chemotherapy and total parental nutrition (TPN). In particular, central venous catheter-related infections are a common cause of bacteraemia and sepsis.

There are three routes by which infection can occur:
- Intraluminal
- Extraluminal
- Haematogenous

Most in-dwelling e.g. vascular catheters are colonized by micro-organisms. The colonizing micro-organisms are usually imbedded in a biofilm layer, they are metabolically active and viable, and they can already be present 24 h after insertion of the catheter.

Organisms causing bloodstream infections generally enter the bloodstream from the skin insertion site or through the connecting hub of the catheter which remains outside the skin but haematogenous seeding and contamination of infused fluids are possible causes as well.

When following the extraluminal route skin organisms migrate from the skin insertion site along the external surface of the catheter, colonizing the distal intravascular tip of the catheter and ultimately causing bloodstream infection. When following the intraluminal route, organisms may be introduced into the hub e.g. by the hands of medical personnel. The subsequent colonization of the internal surface of the catheter may also cause bloodstream infection.

Many clinicians feel reluctant to remove the catheter, because most patients with cuffed tunnelled catheters have exhausted all other options for vascular access.

Health-care institutions purchase millions of intravascular catheters each year. The incidence of CRBSI varies considerably by type of catheter, frequency of catheter manipulation, and patient-related factors (e.g., underlying disease and acuity of illness). Peripheral venous catheters are the devices most frequently used for vascular access. Although the incidence of local or bloodstream infections associated with peripheral venous catheters is usually low, serious infectious complications produce considerable annual morbidity because of the frequency with which such catheters are used. However, the majority of serious catheter-related infections are associated with central venous catheters (CVCs), especially those that are placed in patients in ICUs.

In the ICU, central venous access might be needed for extended periods of time; patients can be colonized with hospital-acquired organisms; and the catheter can be manipulated multiple times per day for the administration of fluids, drugs, and blood products. Moreover, some catheters can be inserted in urgent situations, during which optimal attention to aseptic technique might not be feasible. Certain catheters (e.g., pulmonary artery catheters and peripheral arterial catheters) can be accessed multiple times per day for hemodynamic measurements or to obtain samples for laboratory analysis, augmenting the potential for contamination and subsequent clinical infection.

Specific examples of catheters causing problems are so-called peripherally inserted central catheters (PICCs), midline catheters, and peripheral catheters. A typical PICC, midline, or peripheral catheter contains a thin, flexible shaft, which contains one or more lumens and which terminates at the proximal end with a suitable fitting, such as a hub or other fitting. The primary difference between these three devices is the length of the tubing, with the peripheral catheter being the shortest and the PICC being the longest. The rationale for different lengths is driven by the type and duration of the therapy a patient is to receive.

Hemodialysis catheters are another important class of central venous access catheters. Hemodialysis catheters are commonly multi-lumen catheters in which one lumen is used to carry blood from the body to a dialysis machine, and another lumen returns blood to the body. Central venous access may be attained by puncture of various major blood vessels, including the internal jugular vein, subclavian vein, or femoral vein.

A catheter may further include various accessory components, for example, molded components, over-molded sub-assemblies, connecting fittings such as hubs, extension tubes, and so forth. Various catheter tips designs are known, including stepped tips, tapered tips, over-molded tips and split tips (for multilumen catheters), among others.

Respiratory circuits are another medical device where keeping a sterile environment is a challenge. Due to the inherent moisture and warmth, respiratory circuits provide superb conditions for microbiological growth or colonization. Once colonization has started, the microbiological growth can easily spread to the patient, either airborne or through moisture condensation running down into the patient's lungs, thus risking infections and complications, often resulting in pneumonia.

The problem of respiratory circuit colonization is especially prevalent within breathing tubes. For instance, studies have documented the health risks from colonization of biofilm in endotracheal tubes, which can be so extensive that the walls of the endotracheal tube become slimy and sticky.

Due to the close proximity to the patient's lungs, any microbiological growth in a breathing tube can easily spread to the patient's lungs. Condensed moisture can run down the breathing tube, over the biofilm and into the patient's lungs. Additionally, chunks of the biofilm can actually fall off the breathing tube and into the patient's lungs.

### Disinfection with ultraviolet-C (UVC) light

It is known that microorganisms, such as viruses, bacteria, fungi, protozoa, algae, and so forth can be inactivated (i.e., either killed or prevented from reproducing, e.g., by molecular rearrangement of the microorganisms DNA) using light of various wavelengths, including ultraviolet light of various wavelengths such as ultraviolet-C (UVC) light having a wavelength of 100 to 280 nm, ultraviolet-B (UVB) light having a wavelength 280 to 320 nm, and ultraviolet-A (UVA) light having a wavelength of 320 to 400 nm. For example, UVC light has a very short wavelength and kills bacteria and viruses so well that it is often used to sterilize surfaces. UVB light has also been reported to kill microorganisms.

Several light sources emitting light with the same and/or different wavelengths having bactericide effects could be coupled together in a unit wherein optics could capture and guide the emitted lights into the lumen of a medical device having sterilizing effect. Moreover, a light source could comprise several diodes emitting light with the same and/or different wavelengths having bactericide effects. By this a stronger sterilizing effect could be achieved.

### Other optical devices used for medical diagnostics and treatment

Medical devices such as endoscopes commonly employ light emitting components, such as light sources and light guides, for introducing light into the subject and various coupling designs are available, which readily allow the connection and disconnection of light emitting components to and from the device. For example, couplers and end fittings for optical cables, which allow for efficient coupling of light to and from the optical cables, are presently known in the medical arts including those available from Codman, Fuji, Pentax, Pilling, Storz, and Wolf, among others. Of course other designs, including other unthreaded and threaded designs, including Luer, press fit, and bayonet type couplings, among others, may be employed.

### Prior art

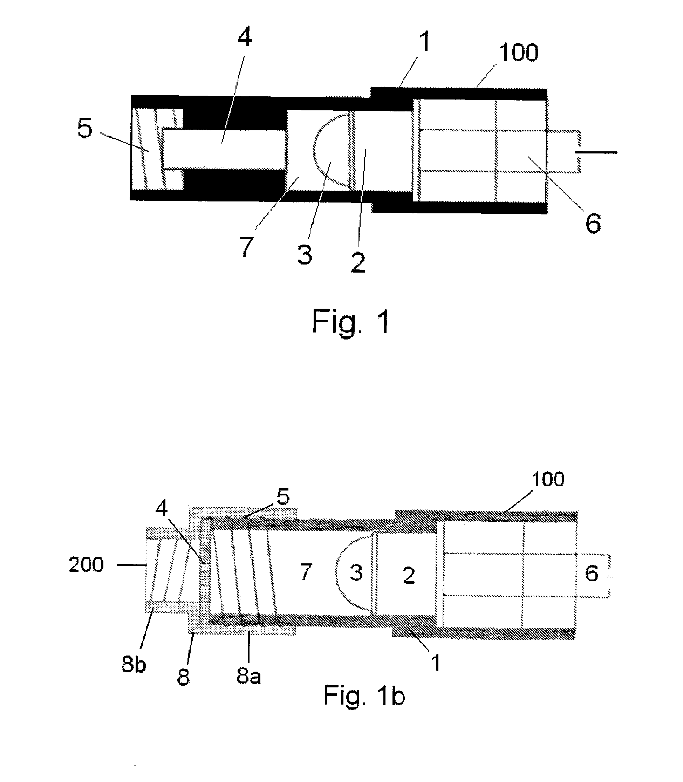

US 2008/0051736 discloses a medical device in the form of an indwelling catheter provided with a light source configured such that light is transmitted from the light source into the catheter shaft or lumen for sterilization purposes. The intention is to sterilize the whole lumen of the catheter which is difficult as this necessitates that the light is guided from the light source through the length of the lumen e.g. this might necessitate that the material which the catheter has been constructed of can transmit light. Further the light source as e.g. seen in figure 1A, 1B and 1C is integrated with the catheter into one assembly. This makes the assembly relatively expensive. Also it requires some dexterity and skills to correctly introduce or integrate the light source into the catheter and extract it i.e. this operation would be difficult for an elder patient. Furthermore, there is a risk of applying too much force during this process thereby breaking or compromising the light source. The configuration of the light source is not actually described in this document and also it is not possible to control whether the light from the light source is actually emitted with the expected effect.

US 2008/0051736 implies that each catheter has its own light source constructed to fit the specific lumen. In contrast, the present invention employs one configured light source which can be coupled to different interfaces or coupling elements which are adapted to fit different catheters. This simplifies the method of sterilizing medical devices such as catheters and, as we will disclose, makes it simpler to couple the light source with the medical device.

US 2007/0176117 discloses a method and an apparatus for sterilizing access sites such as attachment points for various therapeutic and diagnostic medical devices. More particularly, the invention concerns a sterilization apparatus which includes a substantially UV-C transparent closure cap (16). The closure cap (16) is an UV-transparent cap which is attached to the access site; the apparatus for sterilizing the access site is provided with a cap receiving chamber (28) is formed within a capture member (32). Also mounted within the capture member (32) is a source of UV-C radiation for controllably emitting UV-C radiation in a direction towards cap receiving chamber (28). When the apparatus is to be used the closure cap (16) is first attached to the access site and then the assemblage of the UV-C transparent cap (16) and the access site is inserted through an opening (30) formed in the front wall of the housing and the cap (16) is guided into the receiving chamber (28). The manual positioning of the closure cap (16) at the access site complicates the use of the apparatus.

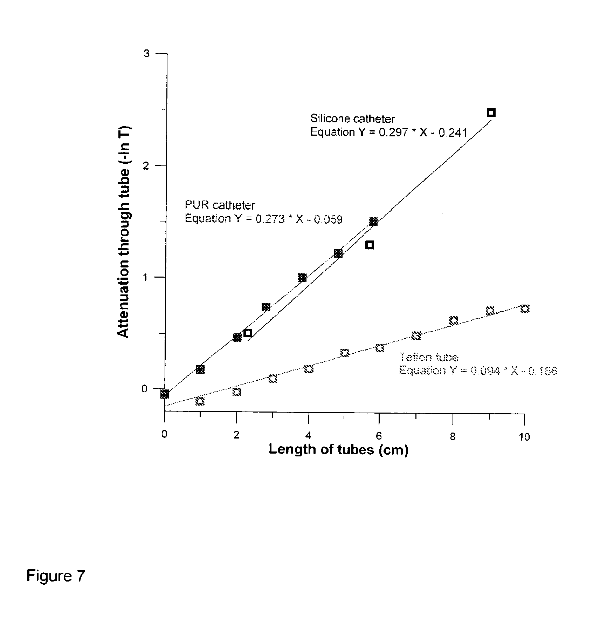

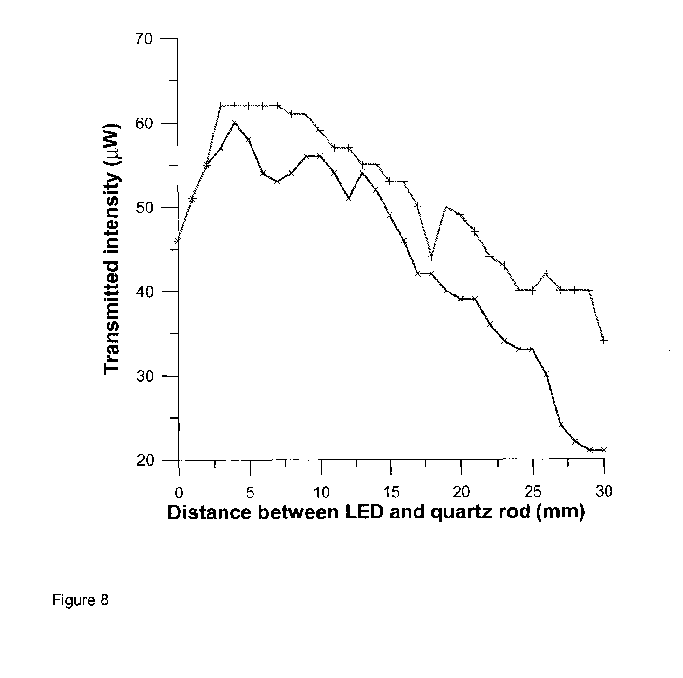

WO 02102421 discloses methods and an apparatus for sterilizing or disinfecting using ultraviolet light or light emitting diodes (LEDs) using one or more several light sources and reflectors. WO 02102421 is primarily directed to a method for sterilizing or disinfecting interior surfaces of a catheter, through the wall of the catheter where the said wall is adapted and thinner allowing transmission of ultraviolet light through a lens and the said wall onto a stagnation zone. The stagnation area is an area where the diameter of the catheter changes and may therefore cause fluid to form eddies in the corners favouring colonization of bacteria. The disinfection apparatus disclosed by WO 02102421 has a clamshell configuration (fig 7A-7C) and comprises a disinfection chamber, wherein a portion of the catheter is placed, the clamshell is closed and the sterilization takes places. The opening (inlet portion) of the catheter can be placed in front of the light source, i.e. along the lumen of the catheter, thereby sterilizing the lumen and the interior surfaces of the catheter (fig 7A- 7C). The catheter can also be placed so that the emitted sterilizing light is perpendicularly to the catheter walls sterilizing a part of the internal and external surfaces of the catheter (Fig 8A-8C). In WO 02102421, the emitted sterilizing light is not guided and focused into the inlet portion of the medical device to be sterilised and the walls of the catheter to be sterilized has to be modified or constructed in such a way to allow UV transmission through the walls. Also, the clamshell configuration of the disinfection apparatus in WO 02102421 will only be suitable for some catheters which fit into the disinfection chamber.

The present invention is primarily directed to be used with medical devices comprising a shaft that contains one or more lumens (e.g., a tube, multilumen extrusion, etc.), which is introduced into a patient for either short or long term residency such as the medical devices described with the technical field.

The present invention is directed 1) to prevent intraluminal routed infections prophylactically and thereby hinder the formation of biofilm and 2) removal of biofilm when biofilm has been formed on the internal surfaces of the lumen of the medical device. The assembly can be used every time the catheter is used e.g. in dialysis hindering the occurrence of a biofilm layer and/or bacteria within the lumen of the catheter in a prophylactic manner.

The object of the present invention is to provide a simply assembly where the light source can easily be coupled to the medical device and stay coupled for as long as it is suitable. If the light source has to be in a use-position for more than a few minutes, the device therefore has to be able to stay in an on-condition without having to be continuously held or influenced by any personnel or the patient. Also, it is the object of the present invention that the light source of the assembly should fit with all standard medical equipment which is in use at hospitals and clinics.

According to the present invention an external light source is placed outside the catheter lumen and connector. With an external source it is achieved that:
- No parts of the catheter lumen are shadowed by the light source i.e. in principle all parts of the catheter and connector part can be swept by germicidal light from the light source.
- No time consuming cleaning and disinfection of the light source housing is necessary between usage of the source (i.e. between treatments and transfer between different lumens of the same catheter.
- It is possible to cool the light source and keep it at its optimal performance.
- No safety problems due to intra-luminal electrical wires and connectors.

### Summary of the invention

The object of the invention is to provide an assembly according to claim 1.

The optical window is placed in front of the light source i.e. between the light source and the medical device. The optical window is transparent to the light from the light source and is an integrated or permanent part of the light source. The protection from the optical window exists both when the light source is joined to the medical device and when the light source is separated from the medical device. The light source and the medical device can be connected and disconnected several times and the upper limit for the number of connections/disconnections is established by the life time of the medical device. The light source can be disinfected and used again with another single-use medical device. The medical device can be an existing and/or commercial device but will normally be adapted according to claim 2 i.e. normally the first connector part is formed and adapted in such a way that it does not cast shadows i.e. provide a shade, into the lumen of the medical device which is to be subjected to the light emitted from the light source. The connector part and also the joint between the connector part and the medical device is formed and adapted in such a way that it does not cast shadows.

One purpose of the separate unit is to be able to combine a standard light source to a standard medical device i.e. the expensive light source and the specialized medical device are coupled together by the use of an inexpensive and disposable coupling device or interface in the form of the separate unit. Also, if a sterile separate unit is taken into use every time the light source as to be connected or reconnected to the medical device, the problem of sterilizing the light source can be reduced.

According to one embodiment the lumen of the separate non-transparent unit and the connector part reaching from the optical window and into the lumen of the medical device has a constant inner or decreasing cross sectional area seen from the inlet towards an in-vivo part of the medical device, and also the inner surfaces does not have any protruding parts or edges which can provide a shade preventing the light from reaching all surfaces of the lumen i.e. the connector part can consist of a conical part such as a female Luer.

According to one embodiment the separate unit contains a sealing device such as a gasket or a O-bearing configured to prevent leakage of fluids from the medical device and into the surrounding or into the light source.

According to one embodiment the medical device is a catheter having an ex-vivo portion. According to this embodiment the surface of the inner lumen of the catheter have a refractive index lower than that of liquid subjected to the lumen during light treatments. Low index refractive surfaces could be achieved either by choosing polymers having low refractive index and/or by coatings the polymers in order to obtain a desired refractive index.

According to one embodiment the light source emits UVC light. According to this embodiment the light source comprises a light-emitting UVC-LED diode.

According to one embodiment the light source can comprise a ball or hemispherical lens system for optimal launch of the UVC light into the narrow tube openings of the medical device.

According to one embodiment the light source (100) has a length of max 50 mm and a diameter of max 15 mm.

According to one embodiment the corresponding connector part is unthreaded, threaded, including Luer, press fit, and/or bayonet type couplings.

According to one embodiment the medical device is an indwelling catheter e.g. selected from peripherally inserted central catheters, midline catheters, peripheral catheters, hemodialysis catheters and venous access ports or peripheral venous catheters or central venous access catheters.

According to another aspect of the invention the invention relates to use of an assembly according to any of the claims 1-12 for sterilizing of the inlet portion of the lumen and surfaces of a medical device.

According to a third aspect of the invention, the invention relates to a method for partly sterilizing a medical device, of an assembly according to any of the claims 1-12, where
- the lumen of the medical device is filled with liquid,
- the light source is joined to the medical device where after the light source emits light having bactericidal effect, such as UV-C light.

According to one embodiment the refractive index n of the liquid in the lumen is higher than the refractive index of water, normally higher than the refractive index of the material of the medical device constituting the inner surfaces of the lumen.

According to one embodiment the refractive index n of the liquid in the lumen is higher than 1.35, normally higher than 1.37, and/or the refractive index n of the material of surfaces of the lumen of the medical device is below 1.34, normally below 1.32.

According to one embodiment the liquid in the lumen is a solution of NaCl or other non-hazardous substance having a refractive index higher than the refractive index of the material constituting the inner surfaces of the medical device, normally the refractive index of the solution should be 0.02 higher than the refractive index of the material constituting the inner surfaces of the medical device.

According to one embodiment the necessary dosing time to obtain a disinfection rate of 99.99 % after preventive UVC light exposure is max 30 minutes.

Embodiments of the invention will now be described with reference to the figures in which:
Figure 1 and 1b each show an embodiment of a light source which can be part of the assembly according to the invention.
Figure 2 shows an assembly according to the invention comprising a catheter and a light source. Figure 2a shows an enlargement of the coupling between the medical device and the light source of figure 2.
Figure 3 shows an embodiment of an interface suitable for a catheter.
Figure 4a shows a female Luer connector part that can be mounted on the medical device. Figure 4b shows a hub with a cylinder opening. Both hubs allows both access of fluids and light (no obstacles)
Figure 5 shows an assembly according to the invention comprising a breathing tube joined with an interface and a light source in a subject.
Figure 6 shows an experimental set up used to evaluate the sterilising effect of UVC light.
Figure 7 shows the attenuation of UVC light through tubes made in materials with different refractive indexes. Attenuation of UVC light through tubes made in materials with different refractive indexes. Showing that the Teflon tube transmits the UVC light (curve with open squares) approximately a factor of 3 more effectively than the catheter tube made in a material of a much higher refractive index (silicone Medcom DuoFlow XL400).
Figure 8 shows the transmitted intensity as a function of the distance between LED and quartzs rod (mm).
Table 1 shows test results demonstrating the disinfection method on biofilm contaminated tube samples
Table 2 shows_the effect of prophylactic treatment of contaminated tubes (20 cm) made of Polyurethane (PUR) and Teflon.

### Detailed description of the invention

The medical device will normally be a catheter, especially a catheter for indwelling use. In medicine a catheter is a tube that can be inserted into a body cavity, duct or vessel. Catheters thereby allow drainage, injection of fluids, diagnostic agents and/or medicine or access by surgical instruments. In most uses a catheter is a thin, flexible tube i.e. a "soft" catheter; in some uses, it is a larger, solid tube i.e. a "hard" catheter.

Several light sources emitting light with the same and/or different wavelengths having bactericide effects could be coupled together in a unit wherein optics could capture and guide the emitted lights into the lumen of a medical device having sterilizing effect. Moreover, a light source could comprise several diodes emitting light with the same and/or different wavelengths having bactericide effects. By this a stronger sterilizing effect could be achieved.

The primary function of conventional catheter hubs is to allow access of liquids such as nutrition, blood, drugs etc. to veins and arteries via the catheter lumen. The hubs or connector parts are not designed for optimal launching of light into a lumen of the medical device. The Luer connector is the standard used for adapting medical devices and join external equipment such as medical machinery and syringes to indwelling implants such as catheters. The Luer connector system consists of a male and female set that provides a leak proof and mechanically secure connection. The Luer connector system is characterized with ~ 6 % conical male end that fits into a conical shaped female part. Because the Luer connector is the standard coupling system used within the medical field the connector system used for the present invention described has to be designed both to satisfy the demands described in the standards and at the same time allow optimal launch of the light. Normally, Luer connectors end up in a small aperture at the distal end which reduces the germicidal effect of the UV light launched into the catheter tube. This reduction of the germicidal effect is caused by:
1. An aperture/opening in the Luer that is smaller than the inner diameter of the tube. This reduces the amount of light emitted from the diode to reach the interior of the tube which results in reduced disinfection efficiency (area ratio between exit hole in Luer and inner tube diameter is less than 1).
2. A small aperture/opening results in a confined ray of light launched into the tube, which do not reach the inner surface of the frontal end of the catheter tube. This also results in reduced disinfection efficiency.

Moreover, the present invention relates to a coupling device which acts as an interface between the light source and catheter hub. This solves two main issues related to catheter use. The first issue deals with the hygiene between UVC treatments. If the light source should be used repeatedly on the same catheter (i.e. same patient) it has to be sterilized before use. If the light source is coupled directly on the catheter hub disinfecting the thread for instance with ethanol would be necessary. This is difficult to do effectively and the light source could actually be a source of contamination. Secondly, all optical surfaces used for diagnostics and treatment have to maintained by cleaning. The optical window is in contact with the catheter hub and the saline solutions in the catheter lumen. It is well-known that precipitation of salt and other compounds on optical windows reduces the transmittance of light which results in reduction of the delivered UVC doses in an uncontrollable manner (not possible to administer a specified UVC dose). Therefore it is important that the optical window separating the LED diode can be cleaned and maintained routinely. The suggested coupling portion solves both problems addressed above. It is meant as a disposable unit that can replaced between UVC treatments. With the coupling portion direct contact between catheter hub and light source is prevented and the light (no contamination source). Removal of the coupling portion exposes the optical window which can cleaned (with ethanol for instance) if necessary.

These and other aspects, embodiments and advantages of the present invention will become immediately apparent to those of ordinary skill in the art upon reading the disclosure to follow.

Figure 1a and 1b show embodiments of lights sources which can be used in connection with the present invention.

The light source 100 shown in figure 1 comprises a housing 1, a source of bactericidal light e.g. an UVC LED (Light Emitting Diode) having a socket 2 and an optical lens 3 e.g. which lens can be flat, ball or hemispherical, an optical window 4 which both guides or at least allows passage of the light to the catheter inlet and protects the entrance to the light source 100 from contaminated fluid, a first connector part 5 comprising an inward thread i.e. female part which can connect either directly to a medical device or to a coupling unit attached which during use is secured to the medical device, and an electrical switch part 6 connecting to an electrical power supply.

The housing 1 provides a watertight encapsulation for the internal parts. The housing 1 has relatively small dimensions i.e. a length less than 10 cm, usually less than 5 cm, and a diameter less than 2 cm, usually less than 1.5 cm. The housing 1 is usually made of a hard polymer or plastic material (for instance acrylic plastic) or thin metal (for instance alumina) which makes it light, strong and easy to form and manufacture. The housing 1 is at the distal end i.e. the end facing the medical device equipped with a first connector part 5 which connector part 5 makes it possible either to link the light source 100 directly to the medical device via a standard opening in the medical device, or to link the light source 100 to an interface (not shown) also called a coupling unit which interface enables a link between the specific medical device and the light source 100. According to the shown embodiment the first connector part 5 is formed as a female thread which will fit into a corresponding male thread on a medical device for instance Luer conical fitting system or an interface.

The UV source shown with this embodiment comprises a diode equipped with a socket and a ball or hemispherical lens 3. The UV source normally emits light in the range between 200 and 300 nm which has a bactericidal effect. The space 7 between the lens/diode system 2, 3 and optical window 4 is occupied by air through which the UV light passes on the way to the optical window 4. The transparent optical window 4 allows the UV light to pass into the lumen of the medical device (inner diameters of the tubes are typically in the range 1-5 mm) which is linked to the light source 100, and additionally the optical window 4 provides a watertight barrier which prevents liquid from the medical device

(typically a high refractive index liquid filled into a catheter lumen) to enter the internal parts of the light source 100 or eventually leak to the surroundings. When applying this light source 100 it is e.g. not necessary to use optical fibres as the 100 can be fastened directly to the medical device.

During the disinfection procedure liquids inside the lumen of the medical device are in close contact with the surfaces of the housing 1 and the surfaces of the optical window 4. Therefore it is necessary to be able to clean these surfaces in order to remove residues that otherwise attenuates the UVC light and thereby reduce the efficiency of the light source 100 as the light source 100 normally is used several times i.e. it might be used several times with one medical device and it might be used with several medical devices. The cleaning can be performed with liquid soap, alcohol such as isopropanol or ethanol or another solvent. Direct cleaning of the light source is difficult as the encapsulated light source cannot be subjected to high temperatures and/or steam etc., and as the light source is intended for multiple uses it will be difficult to assure that no contamination is carried from one medical device to the next.

In order to be able to guarantee sufficient reduction in the number of bacteria at the inlet of the medical device it is necessary that internal and/or external structure(s) of the connector part of the medical device and/or internal and/or external surface(s) of the light source 100, with or without coupling designs, facilitate the emitted light to reach the inlet portion of the lumen of the medical device thereby providing a maximum sterilizing effect. In order to optimize the light entrance into the inlet portion of the lumen of the medical device it must be assured that no part of the light source or of the connector part/hub of the medical device will cast shadows i.e. provide a shade, into the lumen which is to be subjected to the light emitted from the light source. Optimal light conditions will be obtained if the lumen reaching from the optic window 4 and e.g. to first folding of the medical device has a constant inner or increasing cross sectional dimension and also does not have any protruding parts or edges which can provide a shade preventing the light from reaching all surfaces. The inlet portion of the lumen of the medical device and/or the coupling designs can also be coated in any way which facilitates the emitted light to reach the inlet portion of the lumen of the medical device providing a sterilizing effect. The coating can be Teflon, a metal layer, e.g. aluminium layer.

The light source is electrically powered typically a few volts e.g. 6 V through a connection with the electrical switch part 6 which can be connected to a power supply with electrical cords. The power supply can either be placed at a distance or it can comprise batteries placed in connection with i.e. joined directly to the light source 100.

Fig. 1b shows an embodiment comprising a light source 100 having the same or corresponding components as the embodiment of figure 1 a. Components with same or similar function are provided with the same reference number for the different embodiments disclosed in the present application.

Like the light source 100 shown in figure 1 a the embodiment of figure 1b comprises a housing 1, an UV source having a socket 2 and an optical lens 3 e.g. flat, ball or hemispherical, an optical window 4 guiding the light to the catheter inlet, a first connector part 5 which can connect to a medical device, and an electrical switch part 6 connecting to an electrical power supply.

The embodiment of figure 1b is further provided with a separate non-transparent unit 8 functioning as an interface having both a female part 8a with an inward thread which can be connected to the light source 100 and a female part 8b with an inward thread having a smaller diameter and which can be connected to a medical device. This embodiment of the light source and corresponding embodiments provided with an interface placed in fluid tight connection with the optical window 4 is much easier to clean after use. When the light source including a disposable interface or coupling device or separate non-transparent unit is separated from the medical device, the normally disposable interface can be removed and disposed of where after person handling the medical device can clean the smooth surface of the optical window with bactericidal fluids. When only a single smooth surface is to be cleaned then a cleaning method which only comprises sweeping the surfaces with bactericidal fluids such as ethanol can be considered adequate in stead of e.g. subjecting the device to increased heat and/or steam.

In figure 1b the cross sectional area of the opening 200 is less than the cross sectional area of the optical window 4 of the light source 100. But the cross sectional area of the opening 200 can also be equal to the cross sectional area of the optical window 4 of the light source 100.

When the optical window 4 is provided with proper sealing it can be placed between the interface and the light source 100 simply by turning the two units toward each other. This will make it easy to separate the units and clean them individually. If the separate unit 8 is to be used more than once, the simple structure of the unit will normally allow for it to e.g. be autoclaved if a proper material has been chosen. The optical window 4 can be made of UV grade quartz or CaF₂ and will normally be 1-2 mm thick.

In another embodiment the cross sectional area of the optical window 4 and the opening of the light source 200 are the same. The light source 100 can be or is provided with a connector part 5 providing the same inner cross sectional area of the opening of the light source 200 as the optical window 4, facilitating the light emitted from the light source 100 reaching the inlet portion of the lumen of the medical device providing sterilizing effect of all surfaces at the lumen inlet.

The optical window 4 can have any suitable thickness and be placed at any distance from the optical lens 3 facilitating the emitted light to reach the inlet portion of the lumen of the medical device and thereby providing a maximum sterilizing effect. The optimum distance between LED diode or lens system and the tube opening i.e. the distance where a maximum amount of light is transmitted is normally 3-10 mm for the presently known light sources. But any distance providing an optimal transmission of the light is within the scope of this invention. Similarly, the optical window 4 can have a smaller or bigger cross sectional diameter than the optical lens and/or the opening of the light source 200 facilitating the emitted light to reach the inlet portion of the lumen of the medical device providing a sterilizing effect.

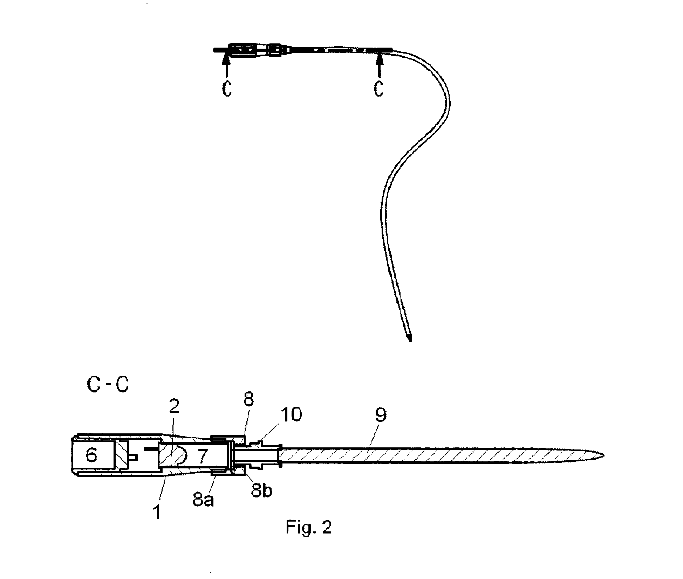

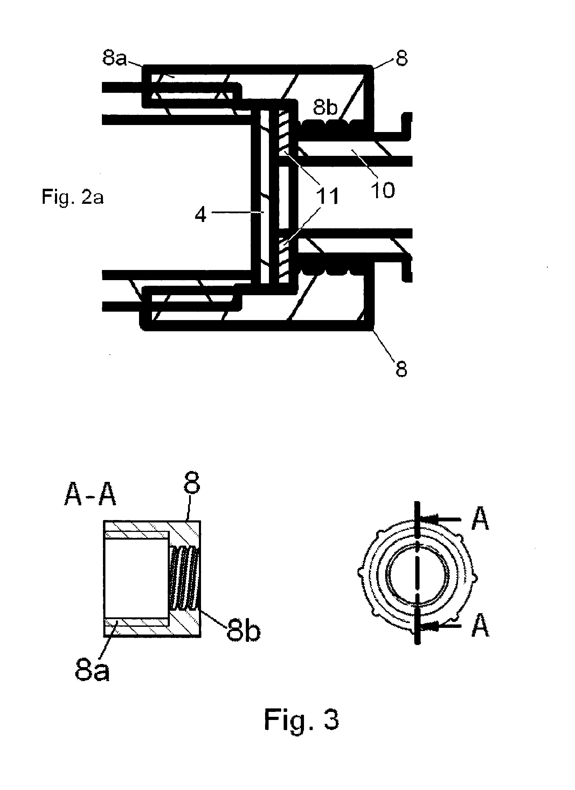

Fig. 2 shows an assembly according to the invention comprising a light source 100 and a separate unit 8 linked to a catheter 9 having an inlet hub i.e. a connector part 10 and an open inner lumen reaching from the optical window 4 of the light source to the first folding of the catheter which open lumen has a constant cross sectional area. In this embodiment the light source 100 is of the same type as the light source 100 shown in figure 1 b.

Firstly, the separate unit 8 is assembled with the light source 100. The light source 100 is screwed on the separate unit 8 until watertight contact is made between a gasket 11 placed in the bottom of the separate unit and the optical window 4 of the light source 100. Fig. 2a shows an enlargement of the separate unit 8 used in fig. 2, where the gasket 11 placed between the optical window 4 and the separate unit 8 is visible. A watertight contact is made between the optical window 4 and the gasket 11 while light can flow through the optical window 4 and through the corresponding central opening of the gasket 11. After the separate unit 8 has been fastened to the light source 100, the separate unit 8 is then screwed on the catheter inlet hub 10 which is also referred to as the connector part.

After the light source 100 has been assembled to the catheter the light treatment e.g. with UV-C light can be initiated. Before joining the assembly, the catheter has been filled with an aqueous solution of for instance sodium chloride for optimal guidance of the light through the connector part 10 and tube lumen. The UV LED diode 2 is powered by a low DC voltage source, e.g. 6 V. Typically a few hundreds microwatts UVC light is emitted from such a diode. The optimal wavelength used for bactericidal purposes is close to 265 nm. An optical path length (1-2 cm) between the diode and edge of the connector part 10 ensures that the inner lumen of the inlet is exposed to UVC light to a degree where sterilization takes place.

The purpose of the invention is not to sterilize or disinfect lumens or surfaces of medical devices which are severely contaminated e.g. due to a very long dwell time. The intention is to use the invention as a preventative mean i.e. by disinfecting the inner surfaces of the connector part and outermost portion of the lumen of the medical device. Therefore preventative light treatments of the entrance of medical devices with the described invention will normally take place right after the medical device has been inserted, and thereafter e.g. be repeated before and after use of the medical device e.g. before and after a hemodialysis session. No contamination of the inner surface of newly inserted catheters is normally expected. If the catheter is contaminated and a biofilm is established intra luminal catheter salvage can be possible using the assembly by administering longer light treatment periods. During handling of the medical devices i.e. opening and closing of inlet hubs by personnel, contact to the patient's skin and exposure from external possible sources of bacteria, such as medical equipment e.g. syringes and tubes, bacteria can penetrate through the connector part and start colonizing the inner lumen. Therefore, it is important to sterilize as much of the proximal end of the medical device immediately after it has been handled in order to prevent biofilm formation, which eventually later on could be spread to other parts such as distal ends of a catheter being in contact with the patient. After the light source 100 has been coupled to the inlet portion of the medical device, e.g. a catheter, which is to be sterilized or disinfected the light source 100 and the catheter might be aligned avoiding bending of the catheter. Although it is possible to sterilize or disinfect Teflon tubes having a moderate bend any bend in the catheter which decreases the emitted light from reaching the inlet portion of the medical device providing sterilizing effect should be avoided.

Fig. 3 shows a separate unit 8 functioning as an interface and having two oppositely placed coupling positions. A first coupling position 8b is to be coupled to a connector part 5 of a light source 100 when the unit is in use and a second coupling position 8a is to be coupled to a connector part 10 of a medical device when the unit is in use.

To achieve optimal disinfection conditions the use of a modified male Luer connector end fitting into a standard female end is preferred. This allows optimal access of the UV light into the lumen of the medical device and satisfies both the need for liquid access and optimal launching of the light into the medical device.

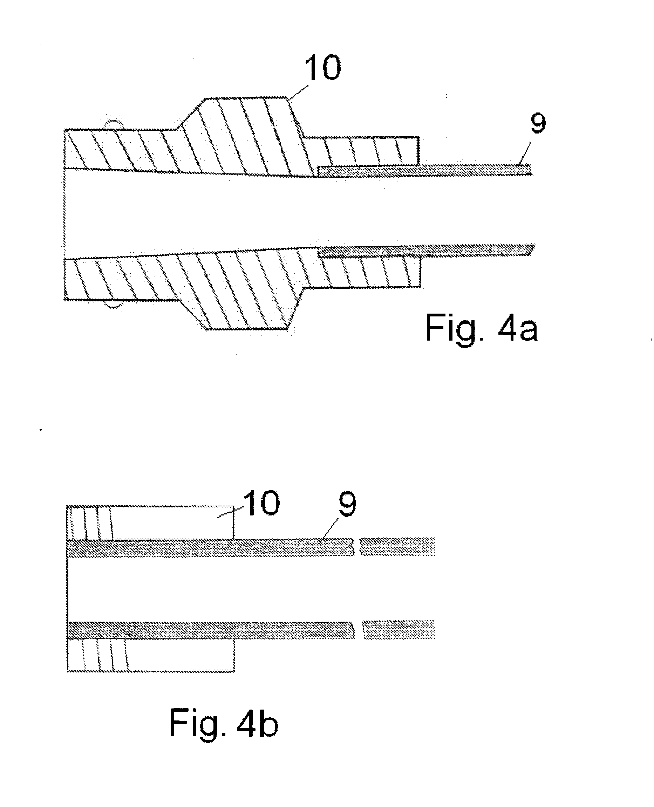

Fig. 4a and 4b show and two embodiments of a connector part 10 attached to a catheter tube 9, where the combined or joined connector part 10 and catheter tube has the same inner cross sectional area as the lumen of the catheter. This can be obtained either by providing the inner surface of the hub i.e. the connector part 10 with a cut-out in the inner surface facing the catheter tube which cut-out is deep enough to contain the cylindrical walls of the catheter tube as shown in fig. 4a or to let the hub i.e. the connector part 10 surround the catheter tube in such a way that the catheter tube is in level with the outer end-surface of the hub as shown in fig. 4b. The connector part 10 is in both embodiments formed with a male thread (illustrated by small bulks on the outer surface at the open end of the catheter in fig. 4a and with lines in fig. 4b). This allows both light to be launched effectively into the tube lumen of the catheter 9 and liquids to be flushed through the lumen of the connector part 10 having a straight cylindrical shape and the same inner diameter as the tube lumen. The connector part 10 can be glued, welded or molded directly at the outer side of the catheter tube i.e. the outer diameter of the tube fits into the inner diameter of the connector part. With no edges and no dead locks between the connector part 10 and the tube lumen no reduction of light will be observed when the light is launched into the lumen. With this design no parts of the connector part 10 is shaded and able to harbour microorganisms due to deadlocks.

Another solution can be to insert and join the tube in a recess of the connector part 10. The connector part 10 can be made of all kinds of polymers or coated polymers, especially of Teflon which has a low refractive index ensuring optimal light propagation. Normally the connector part 10 is moulded.

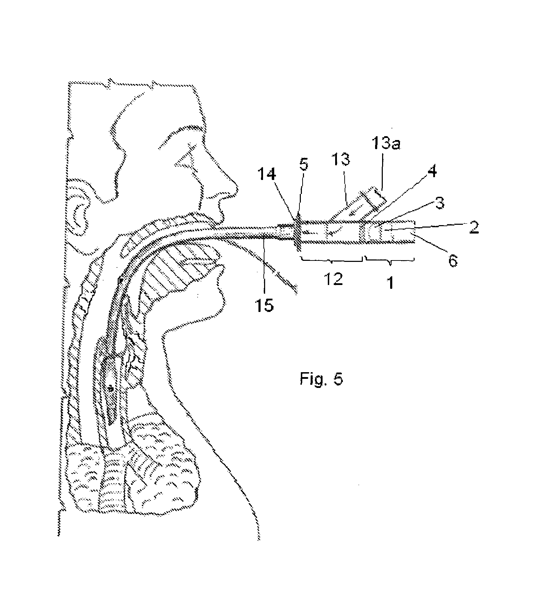

Fig. 5 shows another embodiment of an assembly according to the invention which embodiment comprises a breathing tube 15 having a first end 14 provided with a connector part and a second end inserted in the trachea of a subject, and a light source having a modified interface 12 provided with a corresponding connector part 5. The modified interface 12 is attached to the breathing tube 15 at the first end 14 in extension of the proximal end of the lumen of a breathing tube 15 which is inserted in a subject. In this case the modified interface 12 and the light source might be an integrated unit being inseparable but normally the light source and the modified interface 12 is constructed as two independent parts. The modified interface 12 comprises a by pass 13 with a lumen 13a through which air or other gasses passes back and forth rendering it possible to attach the combined modified interface 12 and light source to the breathing tube 15 permanently. The inner diameter of the lumen of the breathing tube 15 is often bigger than the diameter of catheters, e.g. up to 10 mm and accordingly, the connecter part 5 of the modified interface 12 has a corresponding size and is e.g. unthreaded, threaded, including luer, press fit, and has bayonet type couplings. The combined modified interface 12 and light source when attached to the proximal end of the breathing tube 15 at 14 constitute a relative rigid portion providing sterilization of the modified interface 12 through which air or gasses flows and the proximal lumen of the breathing tube 15. In another embodiment the lumen or a part of the lumen of the breathing tube 15 could be coated with a metal layer, e.g. aluminium, ensuring that the emitted light from the light source is guided to the second end of the breathing tube 15.

### Experiments

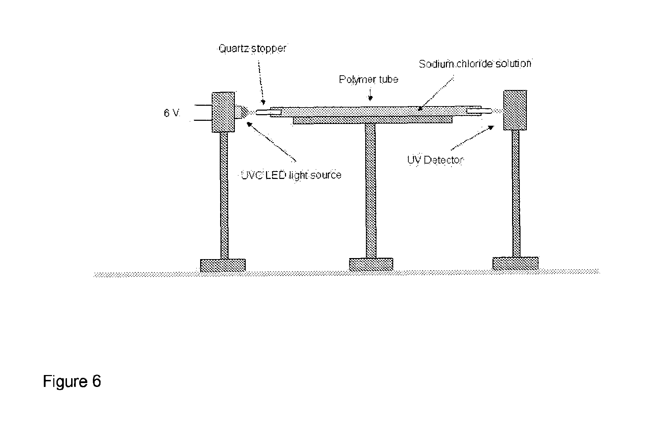

The tubes used for the experiments are made of fluor-ethylene-propylene (FEP Teflon, ADTECH Ltd.), a silicone peritoneal dialysis catheter (Quinton, CurlCath) and the ex-vivo part from a CVC DuoFlow XL400 catheter (Medcom) made in polyurethane (PUR). The tubes have an inner diameter of 3-4 mm and lengths are 20 cm (Teflon) and 10 cm (Teflon and peritoneal catheter). The light propagation through the tubes is favoured if the refractive index of the solution is higher than that of the surface material of the inner part of the polymer tube. The FEP Teflon material is recognized for its low refractive index in the visible range (1.338, see Texloc 2005), which is comparable to that of water. According to the same database the refractive index of the silicone rubber tube is reported to be substantially larger (>1.40). The addition of high concentrations of NaCl raises the refractive index up to 1.40 at 265 nm (Taylor et. al 2004). In this work a 20 % NaCl solution in all the reported measurements are used. For comparison a solution with a 20 % NaCl concentration has a refractive index of 1.368 at visible wavelengths (568 nm; CRC Handbook). It should be noted that the saline solution are reasonable transparent in the UV-VIS spectral range. It is not known due to the apparent lack of data in the UV region for the polymer substances if there is a crossover in refractive indices between the saline solution and the tested polymer materials in the UVC wavelength region. The tubes are after injection of the saline solution sealed with two stops made in UV grade quartz (90 % transmittance) and mounted on top of an adjustable stage (fig. 6). The UVC LED light source (UVTOP 265 nm from Sensor Electronic Technology, Inc.) is fixed in a metal housing for positioning and making electrical contact to a power supply (6 V). Determination of tube transmittances is based on measured light attenuation through tubes in lengths ranging from 1 cm to the full length of the tube samples (10 and 6 cm). The first measurements were on tubes in their full lengths followed by measurements on the same tubes reduced in lengths by cutting sequentially 1 cm away. Visualization of how the light was transmitted and distributed lengthwise in the tubes was done by launching blue light from a HeCd laser (442 nm) through the quartz stopper and into the Teflon tubes which was filled with either pure water or a 20 % saline solution. It was then possible to visualize the effect of liquid light guiding.

A ball lens placed on top of LED diode focuses the light into a small spot specified by the manufacturer to be approximately 1.5-2 mm in diameter at a focal length between 15-20 mm. The angle of the light cone launched into the tube openings is approximately 6 degrees. The total output power from the continuous wave operated LED diodes (electrical current: I_{F}=20 mA) is measured with a UV detector (Blak-Ray, model J-225). The measured total UVC output (~0.25 mW) is close to that specified by the manufacturer at 265 nm with the specified electrical current.

### Procedure for generating pseudomonas biofilm

A peristaltic pump (Masterflex L/H High pressure pump, Cole Parmer Instruments) was used to maintain a constant flow through tubes placed in parallel. The flow rate was maintained at 20 ml/min during all biofilm growth experiments in all tubes (control and UVC treatment). A *Pseudomonas aeruginosa* culture (clinical strain, isolate from a patient with urinary infection, 5 ml 10⁵ CFU/ml in 5% serum bouillon) was diluted in 100 ml nutrient (serum bouillon). This solution was next further diluted in one litre of 0.9% saline solution. During the contamination procedure the pump system was run in four half hour time intervals (total 2 hours duration daily) during three days at a constant temperature (37 °C). With a parallel (or serial) set-up tubes for control measurement and UVC treatments comparable levels of biofilm could be obtained. Retrospectively, the experimental set-up for biofilm formation in tubes/catheters is close to that reported by others (Donlan 2008).

### UVC treatment of tubes with Pseudomonas aeruginosa biofilm and culturing

After biofilm formation the tubes were flushed with sterile water in order to remove the residual bacteria solution from the tube lumen. Next the tubes were filled with a sodium chloride solution (20 % wt. NaCl). The tubes for UVC treatment were then placed in the set-up shown in figure 1. UVC doses ranging from 15 to 300 minutes were applied to the tubes. After UVC treatment tubes (pairs of control and UVC treated) were flushed with sterile water before biofilm was sampled. A brush (pipet brush) was used to remove biofilm from both UVC treated and control tubes. The collected biofilm sample was flushed into a test tube and suspended into 5 ml of nutrient broth and shaken in a Whirley mixer for 2 min. Ten-fold dilutions of each sample were repeatedly made in 0.9 % saline solutions (0.5 ml from the first suspension to 4.5 ml saline). 0.2 ml of each of these dilutions were spread on blood agar plates and incubated aerobically at 37 C for 24 hours. After incubation, the number of colony forming units (CFUs) was determined for the UVC-treated biofilm samples and controls. The counted number of CFUs ranged from < 5 CFU/ml (detection limit - first dilution) to a total of 1.3×10⁹ CFUs/ml. Biofilm samples for viable count determination of both control and UVC treated samples were taken by brushing the entire inner surface (total length). Both samples were suspended in enriched broth solution. The viable count of the control and UVC treated tube samples can be seen in table 1. The level of biofilm contamination in the tubes before exposure to UVC light is seen to be very high.

**Table 1**

| **Material (length/cm)** | Teflon (10) | Teflon (10) | Teflon (10) | Teflon (10) | Teflon (20) | Teflon (20) | Silicone (10) |
|---|---|---|---|---|---|---|---|
| **CFU before UVC exp.** | 3×10⁸ | 7.5×10 ⁶ | 1.5×10 ⁶ | 1.5×10 ⁸ | 1.5×10⁸ | 1.3×10⁹ | 5×10⁵ |
| **UVC dose (min)** | 15 | 30 | 80 | 300 | 30 | 300 | 300 |
| **CFU after UVC exp.** | 50 | 0 | 0 | 0 | 5.5×10⁶ | 0 | 50 |
| **Disinfection rate (%)** | 99.99 | 100 | 100 | 100 | 96 | 100 | 99.99 |

It is expected that the contamination level created here is much higher than that observed in newly inserted catheters. The UVC disinfection unit was also tested using a 10 cm piece of silicone tube (Peritoneal catheter). The silicone tube used in this experiment had a refractory index of approximately 1.45. A disinfection rate of 99.99 % was achieved on this relatively high refractive index catheter tube material after 3 hours of exposure with the light source in form of a UVC LED unit.

### Disinfection of tube lumens with UVC LED exposure

The germicidal effect of the UVC light on *Pseudomonas aeruginosa* biofilm in contaminated tubes is shown in table 1. The number of CFU/ml found on the control tubes varies up to four orders of magnitude ranging from 5×10⁵ - 1.3×10⁹ CFU/ml. Different numbers of bacteria at start and slightly different conditions for growth during the contamination procedure with the flow system are probably the main reason for the differences in number of CFU on the control samples.

Varying treatment times (15 - 300 min) corresponding to doses in the range 0.1-2.1 J (15 × 60 s × 117×10⁻⁶ J/s = 0.1 J) were applied. The efficiency of the UVC light was tested at two different tube lengths (10 and 20 cm). It is observed that relative low doses are required to effectively kill the *Pseudomonas aeruginosa* in 10 cm Teflon tubes (logCFU reduction = 6.78 for a 15 min dose). It is observed that a substantially higher dose is required to obtain the same killing rate for a 20 cm Teflon tube (300 min). This result is supported by the exponential attenuation of the UVC light towards the distal of the tube. From table 1 it is seen, that the intensity at the end of the 20 cm Teflon tube (15.5 %) is approximately a factor of 2.5 weaker than at the end of the 10 cm tube (39.5 %). It is expected then that the exposure time should be increased by the same factor in order to obtain the same disinfection rates as those obtained for the 10 cm tubes (i.e. at least 45 min). Finally a part (10 cm) from a peritoneal catheter made of silicone was contaminated and UVC treated. A reasonable disinfection rate is obtained for this high refractive index material (log CFU reduction = 4.00 i.e. 99.99 %). The applied dose is, however, substantially higher compared to what was necessary for the 10 cm Teflon tubes and the start CFU are smallest of the numbers found on all the tubes. From table 1 it is found that the difference in output intensity between the two tube materials of equal lengths (10 cm) is approximately a factor of 6 (exp[-0.094x 10]/exp[-0.273×10]). We therefore expect that the treatment times should be at least a factor of 6 longer for the silicone/PUR tubes compared to the Teflon tubes in order to obtain comparable disinfection rates.

The sodium chloride concentration for optimal UV light propagation is dependent among other things on the refractive index of the wall material, tube length and the angle of the UV light launched into the tube opening. For 10 cm Teflon tubes we have found that sodium chloride concentrations around 10% are optimal but 0.9-30 weight % NaCl solution can be used and experiments indicate that lower concentrations of NaCl require longer exposure time with the UVC light source. Preferable concentrations of NaCl are 0-10 weight %, 5-10 weight %, 10-15 weight % and 15-20 weight %.

As illustrated in table 1 even a high level of contamination can be reduced substantially if the dose is sufficiently high and/or the catheter material has a low refractive index. Disinfection of a catheter made in materials which are normally used in the clinic, such as silicone (peritoneal catheter) are also possibly even if these tubes are heavily contaminated.

The doses required for preventively disinfecting medical devices such as catheters are expected to be relatively low compared to the doses shown in table 1. The exact doses can be found by clinical trials in which catheters in-vivo are UVC treated, or alternatively can the number of CFU be determined to different dwell times while it is inside the patient.

### UVC Transmittance of Teflon and catheter tubes

Tubes made of different polymer materials are not expected to transmit the UVC light with equal efficiency. Before the transmittance of the different tubes can be determined, it is necessary to measure the exact intensity which is launched into the tubes through the quartz stop, see fig.6. It is observed that part of the UVC light is lost as stray light before it is launched into the tube. Approximately 0.117 mW out of total output power of the diode (0.250 mW) is typically launched into the tube opening. The reflection loss from the quartz stops (10 %) and the exact UVC light intensity launched into the stop can be determined by measurements. It is then possible to determine the transmittance of the tubes (material and saline solution). The measured transmittance data expressed as -In(T) for the various tube materials and lengths is plotted in fig.7. It is observed from the figure that the attenuation, expressed as '-In(T)= a×L' is linear. The loss through the tubes is therefore well described by an exponential function. The attenuation of the light at distance 'L' through the tube can then be expressed as: I = I₀ exp[-axL]. Here 'I₀' is the input intensity after the stop and 'I' is the intensity before leaving the last stop (detector reading corrected for the loss through the last stop). The constant 'a' is a damping parameter which takes into account all possible loss mechanisms such as: absorption in the tube material, light scattering due to the roughness of the tube surfaces and the relative refractive index of the tube and saline solution. If biofilm is present on the inner surface intensity loss due to absorption and scattering from biofilm and cellular components is expected too. It is apparent form the plot that the low refractive material FEP Teflon performs much better than the real catheter material made of silicone and PUR. In the specific case where both tubes are filled with a 20 % NaCl solution the Teflon material transmits 39.4 % and 6.5 % is transmitted through a silicone/PUR tube with equal length (10 cm). The attenuation of the UVC light through the silicone and PUR tubes seems to be comparable (same slope).

It is of interest to know the optimum distance between LED diode - lens system and tube opening where the maximum amount of light is transmitted. Fig.8 illustrates the transmitted intensity in micro-watts as a function of distance between light source and quartz stop. Due to various uncertainties perfect repeat measurements are difficult to obtain and the data points in fig. 8 are seen to fluctuate substantially. The two curves are based on measurements taken at the same tube but in opposite directions. The most important result here is that there is a range of distances where the light from the diode - lens system is most efficiently launched into the tube opening. It is observed from fig. 8 that distances between 3-10 mm are optimal. Increasing the diode - tube opening distance simply results in spreading the light cone from the source. Light is also lost if the lens is to close to the tube opening because the lens diameter is larger than the tube opening.

### Prophylactic UVC treatments

Smaller UVC doses are expected to disinfect tubes with early bacteria contamination than those with established biofilms. If for instance, the UVC treatment of catheter lumens is initiated from the start after catheter placement and repeated after each time the catheter has been used much smaller doses (e.g. treatment times) are expected to be necessary in order to obtain a high degree of disinfection. This prophylactic approach was tested by introducing an 'aseptic breach' which simulated an unforeseen accidental contamination of the catheter lumen. Bacteria (*Pseudomonas aeruginosa*) were cultured in bouillon and injected into Teflon tube lumens in concentrations of 10³ - 10⁴ CFU/ml and kept at 37 °C for 3-5 hours. This contamination level is in the high end of what is expected if an accidental breach caused by an external source (machinery, blood/solutions or humans) should take place. The temperature simulates the body temperature and the time is the period in which the catheter could be expected to be used for medical purposes (dialysis treatment for instance). Contrary disinfection of tubes with established biofilm short UVC treatments is sufficient in the aseptic breach case. The effect of UVC doses corresponding to treatment times between 1 - 30 min was examined. It was found that in all the tests with the UVC device the tube lumen including both the saline solution in the lumen but also the tube walls were disinfected. This goes for both short (10 cm) and longer Teflon tubes (20 cm) and for high concentrations of sodium chloride (10 - 20 %) and at physiological concentrations (0.9 %). In fact in some cases all solution both that scraped from the tube wall and the liquid sampled from the lumen was plated with a zero count as the result (100 % disinfection). Effective UVC treatments within few minutes make the device applicable for clinical purposes.

Table 2 shows the effect of prophylactic treatment of contaminated tubes (20 cm) made of Polyurethane (PUR) and Teflon. An aseptic breach has been introduced the first day injecting *Pseudomonas a.* into the tubes. After 3 hours the tubes were emptied and filled with a 0.9 % saline solution. One tube was used as a reference the three others were UVC treated with different doses corresponding to relative short time periods. Liquid samples were drawn from the tubes (100 µL), diluted, incubated and finally plated. Afterwards the tubes were sealed and maintained for 3 days at 37°C. Samples were drawn again and plated as described above.

**Table 2**

| **Tube** | **1 Day UVC dose min** | **1 Day analysis CFU/ml** | **3 Day analysis CFU/ml** |
|---|---|---|---|
| | 0 (Control) | 1020 | 410 |
| **PUR** | 5 | No growth | No growth |
| | 15 | No growth | No growth |
| | 30 | No growth | No growth |
| | 0 (control) | 2100 | 270 |
| **Teflon** | 1 | No growth | No growth |
| | 5 | No growth | No growth |
| | 20 | No growth | No growth |

## Claims

1. An assembly comprising
- a medical device for transporting fluids having a lumen and a first connector part (10), and
- at least one light source (100) configured to emit light having bactericidal effect, which light source (100) comprises a housing (1), which comprises an optical lens (3), an optical window (4), where said optical window (4) is placed between the optical lens (3) and the medical device, and the light source (100) has a corresponding second connector part (5), and the said optical window (4) is transparent for light emitted from the optical lens (3) allowing the light to the inlet of the lumen of the medical device and protecting the light source from liquid gaining access to the light source (100),
- a separate non-transparent unit (8) being provided with a first coupling part and a second coupling part, where the first coupling part of the separate non-transparent unit during use is joined to the corresponding connector part (5) of the light source (100) and the second coupling part during use is joined to the connector part (10) of the medical device, and
where the first connector part (10) of the medical device and the second corresponding connector part (5) of the light source (100) via the non-transparent unit (8) can be joined such that the light source (100) is placed outside and in extension of the lumen of the medical device before exposure to light.

2. An assembly according to claim 1, wherein the first connector part (10) is formed and adapted in such a way that it does not cast shadows i.e. provide a shade, into the lumen of the medical device which is to be subjected to the light emitted.from the light source.

3. An assembly according to claim 2, wherein the lumen of the separate non-transparent unit (8) and the connector part (10) reaching from the optical window (4) and into the lumen of the medical device has a constant inner or decreasing cross sectional area from the inlet towards an in-vivo part of the medical device, and also does not have any protruding parts or edges which can provide a shade preventing the light from reaching all surfaces of the lumen.

4. An assembly according to claim 1, 2 or 3, wherein the separate unit contains a sealing device such as a gasket (11) or a O-bearing configured to prevent leakage of fluids from the medical device and into the surrounding or into the light source (100).

5. An assembly according to any of the claims 1-4, wherein the medical device is a catheter having an ex-vivo portion.

6. An assembly according to claim 5, wherein the refractive index n of the material of the medical device is below 1.34.

7. An assembly according to any of the claims 1-6, wherein the light source (100) emits UVC light.

8. An assembly according to claim 7, wherein the light source (100) comprises a light-emitting UVC-LED diode (2).

9. An assembly according to claim 8, wherein the light source (100) comprises a ball or hemispherical lens system (3) for optimal launch of the UVC light into the narrow tube openings of the medical device.

10. An assembly according to any of the claims 1-9, wherein the medical device is an indwelling catheter e.g. selected from peripherally inserted central catheters, midline catheters, peripheral catheters, hemodialysis catheters and venous access ports or peripheral venous catheters or central venous access catheters.

11. Use of an assembly according to any of the preceding claims for sterilizing of the inlet portion of the lumen and surfaces of a medical device.

12. A method for partly sterilizing a medical device, of an assembly according to any of the claims 1-10, where
- the lumen of the medical device is filled with liquid,
- the light source (100) is joined to the medical device where after the light source (100) emits light having bactericidal effect, such as UV-C light.

13. A method according to claim 12, wherein the refractive index n of the liquid in the lumen is higher than the refractive index of water.

14. A method according to any of the claims 12-13, wherein the refractive index n of a liquid subjected to the lumen during light treatments is higher than the refractive index of the material of the medical device constituting the surfaces of the lumen.

15. A method according to any of the claims 12-14, wherein the liquid in the lumen is a solution of NaCl or of another non-hazardous substance having a refractive index higher than the refractive index of the material constituting the inner surfaces of the medical device.

## Patentansprüche

1. Vorrichtung umfassend
- eine medizinische Anordnung zum Transportieren von Flüssigkeiten mit einem Lumen und einem ersten Konnektorteil (10), und
- mindestens eine Lichtquelle (100), welche zum Aussenden von Licht mit bakterizider Wirkung ausgebildet ist, welche Lichtquelle (100) ein Gehäuse (1) umfasst, welches eine optische Linse (3), ein optisches Fenster (4) aufweist, wobei das optische Fenster (4) zwischen der optischen Linse (3) und der medizinischen Anordnung angeordnet ist, und die Lichtquelle (100) einen entsprechenden zweiten Konnektorteil (5) aufweist, und das optische Fenster (4) für von der optischen Linse (3) ausgesendetes Licht durchlässig ist, wodurch das Licht zum Einlass des Lumens der medizinischen Anordnung gelassen wird, und die Lichtquelle davor geschützt wird, dass Flüssigkeit zur Lichtquelle (100) Zugang erhält,
- eine getrennte, intransparente Einheit (8), welche mit einem ersten Kopplungsteil und einem zweiten Kopplungsteil ausgestattet ist, wobei das erste Kopplungsteil der getrennten, intransparenten Einheit während des Gebrauchs mit dem entsprechenden Konnektorteil (5) der Lichtquelle (100) verbunden ist, und das zweite Kopplungsteil während des Gebrauchs mit dem Konnektorteil (10) der medizinischen Vorrichtung verbunden ist, und
wobei das erste Konnektorteil (10) der medizinischen Anordnung und das zweite, entsprechende Konnektorteil (5) der Lichtquelle (100) über die intransparente Einheit (8) derart verbunden werden kann, dass die Lichtquelle (100) vor der Lichteinwirkung außerhalb des Lumens der medizinischen Anordnung und in dessen Verlängerung angeordnet ist.

2. Vorrichtung nach Anspruch 1, wobei das erste Konnektorteil (10) derart ausgebildet und angepasst ist, dass es keine Schatten wirft, d.h. keinen Schatten in das Lumen der medizinischen Anordnung spendet, welches dem von der Lichtquelle ausgesendeten Licht ausgesetzt wird.

3. Vorrichtung nach Anspruch 2, wobei das Lumen der getrennten, intransparenten Einheit (8) und das sich von dem optischen Fenster (4) ausgehend in das Lumen der medizinischen Anordnung erstreckende Konnektorteil (10) von dem Einlass bis zu einem in-vivo-Teil der medizinischen Anordnung eine konstante innere oder abnehmende Querschnittsfläche aufweist, sowie auch keine vorspringenden Teile oder Kanten aufweist, welche einen Schatten spenden können, der das Licht verhindert, alle Oberflächen des Lumens zu erreichen.

4. Vorrichtung nach Anspruch 1, 2 oder 3, wobei die getrennte Einheit eine Abdichtungsvorrichtung, wie beispielsweise eine Dichtung (11) oder einen O-Ring, aufweist, welche dazu ausgebildet ist, Leckagen von Flüssigkeiten von der medizinischen Anordnung in die Umgebung oder in die Lichtquelle (100) zu verhindern.

5. Vorrichtung nach einem der Ansprüche 1 bis 4, wobei die medizinische Anordnung ein Katheter mit einem ex-vivo-Teil ist.

6. Vorrichtung nach Anspruch 5, wobei der Brechungsindex n des Materials der medizinischen Anordnung unter 1,34 ist.

7. Vorrichtung nach einem der Ansprüche 1 bis 6, wobei die Lichtquelle (100) UVC-Licht aussendet.

8. Vorrichtung nach Anspruch 7, wobei die Lichtquelle (100) eine Licht aussendende UVC-LED-Diode (2) aufweist.

9. Vorrichtung nach Anspruch 8, wobei die Lichtquelle (100) ein Kugelsystem oder ein halbkugelförmiges Linsensystem (3) für das optimale Einführen des UVC-Lichts in die schmalen Rohröffnungen in der medizinischen Anordnung aufweist.

10. Vorrichtung nach einem der Ansprüche 1 bis 9, wobei die medizinische Anordnung ein Verweilkatheter ist, welches z.B. aus peripher eingesetzten Zentralkathetern, Mittellinienkathetern, peripheren Kathetern, Hämodialyse-kathetern und venösen Portkathetern oder peripheren venösen Kathetern oder venösen Zentralkathetern ausgewählt ist.

11. Verwendung einer Vorrichtung nach einem der vorgehenden Ansprüche zum Sterilisieren des Einlassteils des Lumens und von Oberflächen einer medizinischen Anordnung.

12. Verfahren zum teilweisen Sterilisieren einer medizinischen Anordnung, einer Vorrichtung nach einem der Ansprüche 1 bis 10, wobei
- das Lumen der medizinischen Anordnung mit Flüssigkeit gefüllt wird,
- die Lichtquelle (100) mit der medizinischen Anordnung verbunden wird, wonach die Lichtquelle (100) Licht mit bakterizider Wirkung, wie beispielsweise UV-C-Licht, aussendet.

13. Verfahren nach Anspruch 12, wobei der Brechungsindex n der Flüssigkeit in dem Lumen höher als der Brechungsindex von Wasser ist.

14. Vorrichtung nach einem der Ansprüche 12 bis 13, wobei der Brechungsindex n einer Flüssigkeit, die während Lichtbehandlungen dem Lumen ausgesetzt wird, höher als der Brechungsindex des Materials der medizinischen Anordnung ist, das die Oberflächen des Lumens bildet.

15. Verfahren nach einem der Ansprüche 12 bis 14, wobei die Flüssigkeit in dem Lumen eine Lösung von NaCl oder einer anderen ungefährlichen Substanz ist, welche einen Brechungsindex aufweist, der höher ist als der Brechungsindex des Materials, das die Innenflächen der medizinischen Anordnung bildet.

## Revendications

1. Ensemble comprenant
- un dispositif médical pour le transport de fluides ayant une lumière et une première partie de connecteur (10), et
- au moins une source de lumière (100) configurée pour émettre de la lumière ayant un effet bactéricide, ladite source de lumière (100) comprenant un boîtier (1) qui comprend une lentille optique (3), une fenêtre optique (4), ladite fenêtre optique (4) étant arrangée entre la lentille optique (3) et le dispositif médical, et la source de lumière (100) présentant une deuxième partie de connecteur correspondante (5), et ladite fenêtre optique (4) étant transparente pour la lumière émise de la lentille optique (3) permettant à la lumière à l'entrée de la lumière du dispositif médical et protégeant la source de lumière contre du liquide acquérant l'accès à la source de lumière (100),
- un unité non-transparente séparée (8) étant pourvue d'une première partie d'accouplement et une deuxième partie d'accouplement, lorsque la première partie d'accouplement de l'unité non-transparente séparée lors de l'utilisation est reliée à la partie de connecteur correspondante (5) de la source de lumière (100) et la deuxième partie d'accouplement lors de l'utilisation est reliée à la partie de connecteur (10) du dispositif médical, et
la première partie de connecteur (10) du dispositif médical et la deuxième partie de connecteur correspondante (5) de la source de lumière (100) par l'intermédiaire de l'unité non-transparente (8) pouvant être reliées si bien que la source de lumière (100) est placée à l'extérieur et dans le prolongement de la lumière du dispositif médical avant l'exposition à la lumière.

2. Ensemble selon la revendication 1, dans lequel la première partie de connecteur (10) est formée et conçue de telle manière qu'il ne projette pas d'ombres c'est-à-dire qu'il ne fournit pas une ombre, dans la lumière du dispositif médical qui doit être soumis à la lumière émise à partir de la source de lumière.

3. Ensemble selon la revendication 2, dans lequel la lumière de l'unité non-transparente séparée (8) et la partie de connecteur (10) arrivant de la fenêtre optique (4) et dans la lumière du dispositif médical présente une section intérieure constante ou décroissante de l'entrée vers une partie in-vivo du dispositif médical, et également ne possède pas de parties en saillie ou d'arêtes susceptibles de fournir une ombre empêchant la lumière d'atteindre toutes les surfaces de la lumière.

4. Ensemble selon la revendication 1, 2 ou 3, dans lequel l'unité séparée contient un dispositif d'étanchéité tel qu'un joint d'étanchéité (11) ou un palier O configuré pour empêcher la fuite de fluides à partir du dispositif médical et dans les environnements ou dans la source de lumière (100).

5. Ensemble selon l'une quelconque des revendications 1-4, dans lequel le dispositif médical est un cathéter ayant une partie ex-vivo.

6. Ensemble selon la revendication 5, dans lequel l'indice de réfraction n du matériau du dispositif est inférieur à 1,34.

7. Ensemble selon l'une quelconque des revendications 1 à 6, dans lequel la source de lumière (100) émet de la lumière UV-C.

8. Ensemble selon la revendication 7, dans lequel la source de lumière (100) comprend une diode électroluminescente LED UV-C (2).

9. Ensemble selon la revendication 8, dans lequel la source de lumière (100) comprend une bille ou un système de lentille hémisphérique (3) pour le lancement optimal de la lumière UV-C dans les ouvertures de tubes étroites du dispositif médical.

10. Ensemble selon l'une quelconque des revendications 1 à 9, dans lequel le dispositif médical est un cathéter à demeure p. ex. choisi parmi les cathéters centraux à insertion périphérique, les cathéters de ligne médiane, les cathéters périphériques, les cathéters d'hémodialyse et les ports d'accès veineux ou les cathéters veineux périphériques ou les cathéters d'accès veineux centraux.

11. Utilisation d'un ensemble selon l'une quelconque des revendications précédentes, pour la stérilisation de la partie d'entrée de la lumière et des surfaces d'un dispositif médical.

12. Procédé de stérilisation en partie d'un dispositif médical, d'un ensemble selon l'une quelconque des revendications 1 à 10, où
- la lumière du dispositif médical est remplie de liquide,
- la source de lumière (100) est reliée au dispositif médical, et après la source de lumière (100) émet une lumière ayant un effet bactéricide, telle que la lumière UV-C.

13. Procédé selon la revendication 12, dans lequel l'indice de réfraction n du liquide dans la lumière est supérieur à l'indice de réfraction de l'eau.

14. Procédé selon l'une quelconque des revendications 12 à 13, dans lequel l'indice de réfraction n d'un liquide soumis à la lumière lors du traitement de lumière est supérieur à l'indice de réfraction du matériau du dispositif médical formant les surfaces de la lumière.

15. Procédé selon l'une quelconque des revendications 12 à 14, dans lequel le liquide dans la lumière est une solution de Na Cl ou d'une autre substance non dangereuse ayant un indice de réfraction supérieur à l'indice de réfraction du matériau formant les surfaces intérieures du dispositif médical.
